(19) **Europäisches Patentamt / European Patent Office / Office européen des brevets**

(11) **EP 1 768 749 B1**

(12) **EUROPEAN PATENT SPECIFICATION**

| (45) Date of publication and mention of the grant of the patent: **15.10.2008 Bulletin 2008/42** | (51) Int Cl.: **A61Q 17/04** (2006.01) **A61K 8/26** (2006.01) **A61K 8/81** (2006.01) **A61K 8/27** (2006.01) **A61K 8/29** (2006.01) |
| --- | --- |

(21) Application number: **05761886.0**

(22) Date of filing: **20.06.2005**

(86) International application number: **PCT/EP2005/007368**

(87) International publication number: **WO 2006/005521 (19.01.2006 Gazette 2006/03)**

(54) **AQUEOUS PHOTOPROTECTIVE COMPOSITION COMPRISING HYDROPHILIC METAL OXIDE NANOPIGMENTS AND A VINYLPYRROLIDONE HOMOPOLYMER; USES**

WÄSSRIGE LICHTSCHÜTZENDE ZUSAMMENSETZUNG AUS HYDROPHILEN METALLOXID-NANOPIGMENTEN UND EINEM VINYLPYRROLIDON-HOMOPOLYMER; VERWENDUNGEN

COMPOSITION AQUEUSE PHOTOPROTECTRICE COMPRENANT DES NANOPIGMENTS D'OXYDE METALLIQUE HYDROPHILE ET UN HOMOPOLYMERE DE VINYLPYRROLIDONE ET UTILISATIONS DE CELLE-CI

(84) Designated Contracting States:
**AT BE BG CH CY CZ DE DK EE ES FI FR GB GR HU IE IS IT LI LT LU MC NL PL PT RO SE SI SK TR**

(30) Priority: **13.07.2004 FR 0407828**
**12.08.2004 US 600781 P**

(43) Date of publication of application:
**04.04.2007 Bulletin 2007/14**

(73) Proprietor: **L'ORÉAL**
**75008 Paris (FR)**

(72) Inventors:
• **L'ALLORET, Florence**
**F-75013 Paris (FR)**
• **SIMMONET, Jean-Thierry**
**F-94240 Cachan (FR)**

(74) Representative: **Miszputen, Laurent**
**L'OREAL - D.I.P.I.**
**25-29 Quai Aulagnier**
**92665 Asnières-sur-Seine Cedex (FR)**

(56) References cited:
**EP-A- 0 518 772          WO-A1-02/055046**
**WO-A1-20/04104111     US-A- 6 060 041**
**US-A- 6 146 649**

• **PATENT ABSTRACTS OF JAPAN vol. 2000, no. 11, 3 January 2001 (2001-01-03) & JP 2000 212051 A (OSAKA SHIP BUILDING CO LTD), 2 August 2000 (2000-08-02)**
• **PATENT ABSTRACTS OF JAPAN vol. 2000, no. 19, 5 June 2001 (2001-06-05) & JP 2001 048731 A (POLA CHEM IND INC), 20 February 2001 (2001-02-20)**
• **DATABASE WPI Week 2002 Derwent Publications Ltd., London, GB; AN 2002-114908 XP002316041 & CN 1 308 102 A (UYNA-N) 15 August 2001 (2001-08-15)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present application relates to a photoprotective composition comprising, in a physiologically acceptable medium:

a) at least one aqueous phase,
b) at least **particular** hydrophilic inorganic nanopigments based on metal oxides untreated with an aluminium phosphate,
c) at least one vinylpyrrolidone homopolymer.

It is well known that light radiation with wavelengths of between 280 nm and 400 nm makes possible browning of the human epidermis and that radiation with wavelengths of between 280 nm and 320 nm, known under the name of UV-B radiation, causes skin burns and erythemas which can harm the development of natural tanning; this UV-B radiation must therefore be screened out.

It is also known that UV-A radiation, with wavelengths of between 320 nm and 400 nm, which causes browning of the skin, is capable of leading to a detrimental change in the skin, in particular in the case of sensitive skin or of skin continually exposed to solar radiation. UV-A radiation causes in particular a loss of elasticity of the skin and the appearance of wrinkles, resulting in premature ageing. It promotes the triggering of the erythemal reaction or accentuates this reaction in some subjects and can even be the cause of phototoxic or photoallergic reactions. It is therefore desirable to screen out UV-A radiation as well.

Numerous cosmetic compositions intended for the photoprotection of the skin have been provided to date.

There currently exists on the market various types of sunscreen agents: inorganic particles and organic screening agents. These screening agents must be able to absorb or block harmful solar radiation while remaining innocuous to the user.

Numerous organic sunscreen agents capable of more or less selectively absorbing harmful UV radiation have been provided to date in the field of cosmetics. However, for various reasons, these screening agents are not entirely satisfactory.

This is why attempts are increasingly being made to avoid the use of these organic screening agents while favouring the use of inorganic metal oxide nanoparticles, in particular nano titanium oxides and nano zinc oxides, which also act as sunscreen agents, mainly by scattering/reflecting UV radiation, while introducing greater safety for the user. These nanopigments generally have a mean size for the unit particle of less than 500 nm and preferably of less than 100 nm. The metal oxide nanoparticles generally used in the formulations may be hydrophilic or else may be hydrophobic.

Some types of metal oxide nanoparticles and more particularly those of titanium oxide and those of zinc oxide have a tendency to whiten the skin after application. This phenomenon is undesirable from an aesthetic viewpoint.

In addition to this undesirable whitening phenomenon, metal oxide nanoparticles are generally difficult to formulate in aqueous compositions and more particularly in vehicles of the emulsion type which are the most commonly used in antisun cosmetics. They have a tendency to produce a phenomenon of sedimentation, to form large aggregates and to destabilize the aqueous formulations comprising them.

Provision has already been made, in Application JP 2001-48731, to surface treat inorganic particles with a polyvinylpyrrolidone in order to improve their dispersibility and their stability in aqueous vehicles. However, this document does not disclose metal oxide nanoparticles with a mean size for the unit particle of less than 500 nm and does not make it possible to solve the problem of whitening produced by these nanoparticles on application.

Document WO 02/055046 relates to an emulsion type composition comprising at least on mineral oxide (coated or not) and at least one polyolefin-derived oligomer or polymer, preferably with a succinic termination. The composition are stable and do not whiten the skin.

Application WO 2004/104111 discloses emulsions based on metal oxide micropigments treated with an aluminium phosphate in combination with an organic polymer of the polyvinylpyrrolidone type as dispersing agent.

Furthermore, the Applicant Company, during its research studies, has found that polyvinylpyrrolidones do not make it possible to improve the dispersibility of hydrophobically treated metal oxide nanoparticles in the aqueous phase.

There thus exists a need to seek novel aqueous antisun formulations based on metal oxide nanoparticles for which, on the one hand, the phenomena of whitening on the skin are substantially reduced, indeed even halted, and, on the other hand, a good stability is exhibited (good dispersibility of the nanoparticles in the aqueous phase).

The Applicant Company has discovered, surprisingly and unexpectedly, that this objective could be achieved by using the combination of **particular** hydrophilic metal oxide nanoparticles untreated with an aluminium phosphate and of a vinylpyrrolidone hompolymer.

Thus, a subject-matter of the present invention is a photoprotective composition comprising, in a physiologically acceptable medium:

a) at least one aqueous phase,

b) at least hydrophilic metal oxide nanoparticles untreated with an aluminium phosphate, **the said** nanoparticles having a mean size for the unit particle of less than 500 nm and preferably of less than 100 nm and being treated with at least one coating agent capable of rendering them hydrophilic,

c) at least one vinylpyrrolidone homopolymer.

Another subject-matter of the present invention is the use of at least one vinylpyrrolidone hompolymer in a photoprotective composition comprising at least one aqueous phase and at least hydrophilic metal oxide nanoparticles untreated with an aluminium phosphate for the purpose of reducing the whitening and/or of improving the stability of the said composition (dispersibility of the nanoparticles in the aqueous phase).

The term "photoprotective composition" is understood to mean a composition capable of screening out UV radiation, in particular solar radiation.

The term "physiologically acceptable medium" is understood to mean a nontoxic medium capable of being applied to the skin, lips, hair, eyelashes, eyebrows or nails. The composition of the invention can constitute in particular a cosmetic or dermatological composition.

The term "nanoparticles" is understood to mean particles for which the mean size of the unit particles is less than 100 nm.

The term "hydrophilic" is understood to mean particles which, introduced into an aqueous phase at 25°C, at a concentration by weight of 1%, make it possible to obtain a solution which is macroscopically homogeneous to the naked eye.

The hydrophilic metal oxide nanoparticles used in the present invention are powders composed of particles having a mean size for the unit particle preferably of between 10 nm and 100 nm and preferentially between 15 nm and 50 nm.

The metal oxides forming these nanoparticles are preferably chosen from titanium oxides, zinc oxides or their mixtures

The treated metal oxide nanoparticles are generally subjected to one or more surface treatments of a chemical, electronic, mechanochemical and/or mechanical nature with at least one compound capable of rendering them hydrophilic, such as those described, for example, in Cosmetics & Toiletries, February 1990, Vol. 105, pp. 53-64. Mention may be made, for example, of amino acids, $C_1$-$C_5$ alkanolamines, silicon oxides (silica), metal oxides, such as alumina, sodium hexametaphosphate or glycerol or their mixtures.

Mention may be made, among the treated titanium oxide nanoparticles which can be used according to the invention, of the titanium oxide nanoparticles treated with at least one coating agent, such as:

- silica and alumina, such as the products "Microtitanium Dioxide MT 500 SA", and "Microtitanium Dioxide MT 100 SA" from Tayca and the products "Tioveil Fin", "Tioveil OP", "Tioveil MOTG" and "Tioveil IPM" from Tioxide, the product "Mirasun TIW 60" from Rhodia, the product "Sunveil PW-6030A-20" from CCIC or the product "Solaveil CT-10W" from Uniquema;
- sodium hexametaphosphate, such as the product "Microtitanium dioxide MT 150W" from Tayca,
- alumina and glycerol, such as the product "UVT-M212" from Kemira,
- alumina, silica and alginic acid, such as "Microtitanium dioxide MT 100 AQ" from Tayca.

Mention may also be made of coated mixtures of titanium dioxide and of zinc dioxide, such as that coated with alumina, with silica and with glycerol, for example the product "M 211" sold by Kemira.

Preference is very particularly given to titanium oxide nanoparticles, whether amorphous or in crystalline (rutile and/or anatase) form.

The hydrophilic metal oxide nanoparticles in accordance with the invention are preferably present in the compositions according to the invention in proportions ranging from 0.5 to 30% by weight, with respect to the total weight of the composition, and preferably ranging from 1 to 25% by weight, with respect to the total weight of the composition.

Mention may be made, as examples of vinylpyrrolidone homopolymers which can be used according to the invention; of the following polymers:

| Commercial name | Number-average molar mass (g/mol) | Supplier |
|---|---|---|
| Luviskol K 17 Powder | 2500 | BASF |
| Kollidon 17 PF | 2500 | BASF |
| Kollidon 12 PF | 1300 | BASF |

(continued)

| Commercial name | Number-average molar mass (g/mol) | Supplier |
|---|---|---|
| Kollidon 30 | 10 000 | BASF |
| Polyvinylpyrrolidone K 60 Solution | 160 000 | Fluka |
| Kollidon 90 | 360 000 | BASF |

According to a particularly preferred form of the invention, the vinylpyrrolidone hompolymers in accordance with the invention have a molar mass of less than 20 000 g/mol and better still of less than 10 000 g/mol.

The concentration by weight of vinylpyrrolidone homopolymer in the composition preferably varies from 0.01% to 10%, more preferably from 0.1% to 5% and better still from 0.2% to 2.5%.

The ratio by weight of the metal oxide nanoparticles to the polyvinylpyrrolidone preferably varies from 1 to 30, more preferably from 5 to 20 and particularly from 8 to 12.

The metal oxide nanoparticles can be brought into contact with a polyvinylpyrrolidone directly during the formulation of the composition, for example by considering an aqueous phase comprising the particles and the polymer introduced at the end of formulation. The metal oxide nanoparticles can also be pretreated with the polyvinylpyrrolidone before the introduction into a composition; this pretreatment can be carried out by dispersing the particles in an aqueous polyvinylpyrrolidone solution, followed by evaporation of the water, in order to recover a powder composed of nanoparticles coated with a polymer.

According to a preferred form of the invention, the ionic strength of the aqueous phase of the composition, without the metal oxide, is less than 0.1 mol/l. The ionic strength is determined using a CDM 230 conductivity meter (Meterlab) resulting in the conductivity of the medium, a parameter related to the ionic strength by the following relationship:

$$\text{Ionic strength (mol/l)} = 0.1 \times \text{Conductivity (Siemens/cm)} + 0.19$$

Furthermore, the compositions in accordance with the invention can comprise other additional organic photoprotective agents active in the UV-A and/or UV-B region which are water-soluble or fat-soluble or else insoluble in the cosmetic solvents commonly used.

The additional organic photoprotective agents are chosen in particular from cinnamic derivatives; anthranilates; salicylic derivatives; dibenzoylmethane derivatives; camphor derivatives; benzophenone derivatives; β,β-diphenylacrylate derivatives; triazine derivatives; benzotriazole derivatives; benzalmalonate derivatives; benzimidazole derivatives; imidazolines; bis-benzoazolyl derivatives, as disclosed in Patents EP 669 323 and US 2 463 264; p-aminobenzoic acid (PABA) derivatives; methylenebis(hydroxyphenylbenzotriazole) derivatives, as disclosed in Applications US 5 237 071, US 5 166 355, GB 2 303 549, DE 197 26 184 and EP 893 119; benzoxazole derivatives, as disclosed in Patent Applications EP 0 832 642, EP 1 027 883, EP 1 300 137 and DE 10162844; screening polymers and screening silicones, such as those disclosed in particular in Application WO 93/04665; dimers derived from α-alkylstyrene, such as those disclosed in Patent Application DE 19855649; 4,4-diarylbutadienes, as disclosed in Applications EP 0 967 200, DE 19746654, DE 19755649, EP-A-1 008 586, EP 1 133 980 and EP 133 981; and their mixtures.

Mention may be made, as examples of additional organic photoprotective agents, of those denoted below under their INCI names:

Cinnamic derivatives:

Ethylhexyl Methoxycinnamate, sold in particular under the trade name "Parsol MCX" by Hoffmann-LaRoche,
Isopropyl Methoxycinnamate,
Isoamyl Methoxycinnamate, sold under the trade name "Neo Heliopan E 1000" by Haarmann and Reimer,
Cinoxate,
DEA Methoxycinnamate,
Diisopropyl Methyl Cinnamate,
Glyceryl Ethylhexanoate Dimethoxycinnamate.

Dibenzoylmethane derivatives:

Butyl Methoxydibenzoylmethane, sold in particular under the trade name "Parsol 1789" by Hoffmann-LaRoche, Isopropyl Dibenzoylmethane.

para-Aminobenzoic acid derivatives:

PABA,
Ethyl PABA,
Ethyl Dihydroxypropyl PABA,
Ethylhexyl Dimethyl PABA, sold in particular under the name "Escalol 507" by ISP,
Glyceryl PABA,
PEG-25 PABA, sold under the name "Uvinul P25" by BASF.

Salicylic derivatives:

Homosalate, sold under the name "Eusolex HMS" by Rona/EM Industries,
Ethylhexyl Salicylate, sold under the name "Neo
Heliopan OS" by Haarmann and Reimer,
Dipropyleneglycol Salicylate, sold under the name "Dipsal" by Scher,
TEA Salicylate, sold under the name "Neo Heliopan TS" by Haarmann and Reimer.

β,β-Diphenylacrylate derivatives

Octocrylene, sold in particular under the trade name "Uvinul N539" by BASF,
Etocrylene, sold in particular under the trade name "Uvinul N35" by BASF.

Benzophenone derivatives:

Benzophenone-1, sold under the trade name "Uvinul 400" by BASF,
Benzophenone-2, sold under the trade name "Uvinul D50" by BASF,
Benzophenone-3 or Oxybenzone, sold under the trade name "Uvinul M40" by BASF,
Benzophenone-4, sold under the trade name "Uvinul MS40" by BASF,
Benzophenone-5,
Benzophenone-6, sold under the trade name "Helisorb 11" by Norquay,
Benzophenone-8, sold under the trade name "Spectra-Sorb UV-24" by American Cyanamid,
Benzophenone-9, sold under the trade name "Uvinul DS-49" by BASF,
Benzophenone-12, n-Hexyl 2-(4-diethylamino-2-hydroxybenzoyl)benzoate.

Benzylidenecamphor derivatives:

3-Benzylidene camphor, manufactured under the name "Mexoryl SD" by Chimex,
4-Methylbenzylidene camphor, sold under the name "Eusolex 6300" by Merck,
Benzylidene Camphor Sulfonic Acid, manufactured under the name "Mexoryl SL" by Chimex,
Camphor Benzalkonium Methosulfate, manufactured under the name "Mexoryl SO" by Chimex,
Terephthalylidene Dicamphor Sulfonic Acid, manufactured under the name "Mexoryl SX" by Chimex,
Polyacrylamidomethyl Benzylidene Camphor, manufactured under the name "Mexoryl SW" by Chimex.

Phenylbenzimidazole derivatives:

Phenylbenzimidazole Sulfonic Acid, sold in particular under the trade name "Eusolex 232" by Merck,
Disodium Phenyl Dibenzimidazole Tetrasulfonate, sold under the trade name "Neo Heliopan AP" by Haarmann and Reimer.

Phenylbenzotriazole derivatives:

Drometrizole Trisiloxane, sold under the name "Silatrizole" by Rhodia Chimie,
Methylene Bis-Benzotriazolyl Tetramethylbutylphenol, sold in the solid form under the trade name "Mixxim BB/100" by Fairmount Chemical or in the micronized form in aqueous dispersion under the trade name "Tinosorb M" by Ciba Specialty Chemicals.

Triazine derivatives:

Bis-Ethylhexyloxyphenol Methoxyphenyl Triazine, sold under the trade name "Tinosorb S" by Ciba-Geigy, Ethylhexyl triazone, sold in particular under the trade name "Uvinul T150" by BASF,
Diethylhexyl Butamido Triazone, sold under the trade name "Uvasorb HEB" by Sigma 3V,
2,4,6-Tris(dineopentyl 4'-aminobenzalmalonate)-s-triazine,
2,4,6-Tris(diisobutyl 4'-aminobenzalmalbnate)-s-triazine.

Anthranilic derivatives:

Menthyl anthranilate, sold under the trade name "Neo Heliopan MA" by Haarmann and Reimer.

Imidazoline derivatives:

Ethylhexyl Dimethoxybenzylidene Dioxoimidazoline Propionate.

Benzalmalonate derivatives:

Polyorganosiloxanes with benzalmalonate functional groups, such as Polysilicone-15, sold under the trade name "Parsol SLX" by Hoffmann-LaRoche.

4,4-Diarylbutadiene derivatives:

1,1-Dicarboxy(2,2'-dimethylpropyl)-4,4-diphenyl-butadiene.

Benzoxazole derivatives:

2,4-Bis[5-1(dimethylpropyl)benzoxazol-2-yl-(4-phenyl)-imino]-6-(2-ethylhexyl)imino-1,3,5-triazine, sold under the name Uvasorb K2A by Sigma 3V;

and their mixtures.

The preferred additional organic photoprotective agents are chosen from:

Ethylhexyl Methoxycinnamate,
Ethylhexyl Salicylate,
Homosalate,
Octocrylene,
Phenylbenzimidazole Sulfonic Acid,
Benzophenone-3,
Benzophenone-4,
Benzophenone-5,
n-Hexyl 2-(4-diethylamino-2-hydroxybenzoyl)benzoate,
4-Methylbenzylidene camphor,
Terephthalylidene Dicamphor Sulfonic Acid,
Disodium Phenyl Dibenzimidazole Tetrasulfonate,
Methylene Bis-benzotriazolyl Tetramethylbutylphenol,
Bis-Ethylhexyloxyphenol Methoxyphenyl Triazine,
Ethylhexyl triazone,
Diethylhexyl Butamido Triazone,
2,4,6-Tris(dineopentyl 4'-aminobenzalmalonate)-s-triazine,
2,4,6-Tris(diisobutyl 4'-aminobenzalmalonate)-s-triazine,
Drometrizole Trisiloxane,
Polysilicone-15,
1,1-Dicarboxy(2,2'-dimethylpropyl)-4,4-diphenyl-butadiene,
2,4-Bis[5-1(dimethylpropyl)benzoxazol-2-yl-(4-phenyl)-imino]-6-(2-ethylhexyl)imino-1,3,5-triazine, and their mixtures.

The aqueous compositions of the invention can be provided in all the forms generally used for a topical application, in particular in the form of an oil-in-water emulsion (direct emulsion), water-in-oil emulsion (inverse emulsion), water-in-oil-in-water emulsion (multiple emulsion) or also of an aqueous gel.

The compositions of the invention can comprise all the additives commonly used in cosmetics and will find applications in the care and makeup field and in the field of antisun products.

The additional photoprotective agents are generally present in the compositions according to the invention in proportions ranging from 0.01 to 20% by weight, with respect to the total weight of the composition, and preferably ranging from 0.1 to 10% by weight, with respect to the total weight of the composition.

The aqueous compositions in accordance with the present invention can additionally comprise conventional cosmetic adjuvants chosen in particular from fatty substances, organic solvents, ionic or nonionic and hydrophilic or lipophilic thickening agents, softening agents, humectants, opacifiers, stabilizers, emollients, silicones, antifoaming agents, fragrances, preservatives, anionic, cationic, nonionic, zwitterionic or amphoteric surfactants, active principles, fillers, polymers, propellants, basifying or acidifying agents or any other ingredient commonly used in the cosmetics and dermatological field.

The fatty substances can be composed of an oil or a wax or their mixtures. The term "oil" is understood to mean a compound which is liquid at ambient temperature. The term "wax" is understood to mean a compound which is solid or substantially solid at ambient temperature and which has a melting point generally of greater than 35°C.

Mention may be made, as oils, of mineral oils (liquid paraffin); vegetable oils (sweet almond oil, macadamia oil, blackcurrant seed oil or jojoba oil); synthetic oils, such as perhydrosqualene, fatty alcohols, fatty amides (such as isopropyl lauroyl sarcosinate, sold under the name of "Eldew SL-205" by Ajinomoto), fatty acids or esters (such as the benzoate of $C_{12}$-$C_{15}$ alcohols sold under the trade name "Finsolv TN" or "Witconol TN" by Witco, octyl palmitate, isopropyl lanolate, triglycerides, including those of capric/carprylic acids, or the dicaprylyl carbonates sold under the name "Cetiol CC" by Cognis), or oxyethylenated or oxypropylenated fatty esters and ethers; silicone oils (cyclomethicone, polydimethylsiloxanes or PDMSs) or fluorinated oils; or polyalkylenes.

Mention may be made, as waxy compounds, of paraffin wax, carnauba wax, beeswax or hydrogenated castor oil.

Mention may be made, among organic solvents, of lower alcohols and polyols. The latter can be chosen from glycols and glycol ethers, such as ethylene glycol, propylene glycol, butylene glycol, dipropylene glycol or diethylene glycol.

Mention may be made, as hydrophilic thickening agents, of carboxyvinyl polymers, such as Carbopols (carbomers) and Pemulens (acrylate/$C_{10}$-$C_{30}$ alkyl acrylate copolymer); polyacrylamides, such as, for example, the crosslinked copolymers sold under the names Sepigel 305 (CTFA name: polyacrylamide/C13-14 isoparaffin/Laureth 7) or Simulgel 600 (CTFA name: acrylamide/sodium acryloyldimethyltaurate copolymer/isohexadecane/polysorbate 80) by Seppic; polymers and copolymers of 2-acrylamido-2-methylpropanesulphonic acid, optionally crosslinked and/or neutralized, such as the poly(2-acrylamido-2-methylpropanesulphonic acid) sold by Hoechst under the trade name "Hostacerin AMPS" (CTFA name: ammonium polyacryldimethyltauramide); cellulose derivatives, such as hydroxyethylcellulose; polysaccharides and in particular gums, such as xanthan gum; and their mixtures.

Mention may be made, as lipophilic thickening agents, of synthetic polymers, such as the poly($C_{10-30}$ alkyl acrylate) sold under the name "Doresco IPA 13-1" by Landec, or also modified clays, such as hectorite and its derivatives, for example the products sold under the Bentone names.

Mention may be made, among the active principles, of:

- vitamins (A, C, E, K, PP, and the like) and their derivatives or precursors, alone or as mixtures,
- agents for combating pollution and/or free radicals;
- depigmenting agents and/or propigmenting agents;
- antiglycation agents;
- soothing agents;
- NO-synthase inhibitors;
- agents which stimulate the synthesis of dermal or epidermal macromolecules and/or which prevent their decomposition;
- agents which stimulate the proliferation of fibroblasts;
- agents which stimulate the proliferation of keratinocytes;
- muscle relaxants;
- tightening agents;
- mattifying agents;
- keratolytic agents;
- desquamating agents;
- moisturizing agents;
- antiinflammatories;
- agents which act on the energy metabolism of the cells;
- insect repellents;
- substance P or CRGP antagonists;
- agents for combating hair loss and/or for promoting hair regrowth;
- antiwrinkle agents.

Of course, a person skilled in the art will take care to choose the optional additional compound or compounds mentioned above and/or their amounts so that the advantageous properties intrinsically attached to the compositions in accordance with the invention are not, or not substantially, detrimentally affected by the envisaged addition or additions.

The compositions according to the invention can be prepared according to techniques well known to a person skilled in the art. They can in particular be provided in the form of a simple or complex emulsion (O/W, W/O, O/W/O or W/O/W), such as a cream, a milk or a cream gel; in the form of an aqueous gel; or in the form of a lotion. They can optionally be packaged in an aerosol and be provided in the form of a foam or spray.

Preferably, the compositions according to the invention are provided in the form of an oil-in-water or water-in-oil emulsion.

The emulsions generally comprise at least one emulsifier chosen from amphoteric, anionic, cationic or nonionic emulsifiers; used alone or as a mixture. The emulsifiers are appropriately chosen according to the emulsion to be obtained (W/O or O/W).

Mention may be made, as emulsifying surfactants which can be used for the preparation of the W/O emulsions, of, for example, sorbitan alkyl esters or ethers, glycerol alkyl esters or ethers or sugar alkyl esters or ethers; or silicone surfactants, such as dimethicone copolyols, for example the mixture of cyclomethicone and of dimethicone copolyol sold under the name "DC 5225 C" by Dow Corning, and alkyl dimethicone copolyols, for example lauryl methicone copolyol, sold under the name "Dow Corning 5200 Formulation Aid" by Dow Corning, cetyl dimethicone copolyol, for example the product sold under the name Abil EM 90® by Goldschmidt, and the mixture of cetyl dimethicone copolyol, of polyglyceryl (4 mol) isostearate and of hexyl laurate sold under the name Abil WE 09 by Goldschmidt. One or more coemulsifiers which can advantageously be chosen from the group consisting of polyol alkyl esters can also be added thereto. Mention may in particular be made, as polyol alkyl esters, of polyethylene glycol esters, such as PEG-30 dipolyhydroxystearate, for example the product sold under the name Arlacel P135 by ICI.

Mention may be made, as glycerol and/or sorbitan esters, for example, of polyglyceryl isostearate, such as the product sold under the name Isolan GI 34 by Goldschmidt; sorbitan isostearate, such as the product sold under the name Arlacel 987 by ICI; the isostearate of sorbitan and of glycerol, such as the product sold under the name Arlacel 986 by ICI, and their mixtures.

Mention may be made, for the O/W emulsions, of, for example, as emulsifiers, nonionic emulsifiers, such as oxyalkylenated (more particularly polyoxyethylenated) esters of fatty acids and of glycerol; oxyalkylenated esters of fatty acids and of sorbitan; oxyalkylenated (oxyethylenated and/or oxypropylenated) esters of fatty acids, such as the PEG-100 stearate/glyceryl stearate mixture sold, for example, by ICI under the name Arlacel 165; oxyalkylenated (oxyethylenated and/or oxypropylenated) ethers of fatty alcohols; sugar esters, such as sucrose stearate; fatty alcohol and sugar ethers, in particular alkyl polyglucosides (APG), such as decyl glucoside and lauryl glucoside, sold, for example, by Henkel under the respective names Plantaren 2000 and Plantaren 1200, cetearyl glucoside, optionally as a mixture with cetearyl alcohol, sold, for example, under the name Montanov 68 by Seppic, under the name Tegocare CG90 by Goldschmidt and under the name Emulgade KE3302 by Henkel, and arachidyl glucoside, for example in the form of the mixture of arachidyl and behenyl alcohols and of arachidyl glucoside sold under the name Montanov 202 by Seppic. According to a specific embodiment of the invention, the mixture of the alkyl polyglucoside as defined above with the corresponding fatty alcohol can be in the form of a self-emulsifying composition, as disclosed, for example, in the document WO-A-92/06778.

When an emulsion is involved, the aqueous phase of the latter can comprise a nonionic vesicular dispersion prepared according to known processes (Bangham, Standish and Watkins, J. Mol. Biol., 13, 238 (1965), FR 2 315 991 and FR 2 416 008).

The compositions according to the invention are applied in a large number of treatments, in particular cosmetic treatments, of the skin, lips and/or hair, including the scalp, in particular for the protection and/or the care of the skin, lips and/or hair and/or for making up the skin and/or lips.

Another subject-matter of the present invention is composed of the use of the compositions according to the invention as defined above for the manufacture of products for the cosmetic treatment of the skin, lips, nails, hair, eyelashes, eyebrows and/or scalp, in particular care products, sun protection products and make-up products.

The cosmetic compositions according to the invention can, for example, be used as care and/or sun protection product for the face and/or body with a liquid to semi-liquid consistency, such as lotions, milks, relatively smooth creams, gels or cream gels. They can optionally be packaged in an aerosol and be provided in the form of a foam or spray.

The cosmetic compositions according to the invention can, for example, be used as make-up product.

The compositions according to the invention in the form of vaporizable fluid lotions in accordance with the invention are applied to the skin or the hair in the form of fine particles using pressurization devices. The devices in accordance with the invention are well known to a person skilled in the art and comprise non-aerosol pumps or "atomizers", aerosol containers comprising a propellant and aerosol pumps using compressed air as propellant. The latter are disclosed in Patents US 4 077 441 and US 4 850 517 (forming an integral part of the content of the description).

The compositions packaged in an aerosol in accordance with the invention generally comprise conventional propellants, such as, for example, hydrofluorinated compounds, dichlorodifluoromethane, difluoroethane, dimethyl ether, isobutane, n-butane, propane or trichlorofluoromethane. They are preferably present in amounts ranging from 15 to 50% by weight, with respect to the total weight of the composition.

The concrete but in.no way limiting examples illustrating the invention will now be given.

**Examples:**

**Examples 1 to 4: Aqueous dispersions comprising 20% as AM of hydrophilic titanium oxide nanoparticles (Mirasun TiW 60) in the presence of 2.2% of polymer, at pH 5**

The polymers used are as follows:

A: Polyacrylic acid, 2000 g/mol (outside the invention)
B: Anhydride and diisobutylene copolymer in the sodium salt form supplied under the name of "Orotan 731 DP" by Röhm & Haas (outside the invention)
C: Polyvinylpyrrolidone with a mass of 2500 g/mol, supplied under the name of "Luviskol K17 Powder" from BASF (invention)

**1) Dispersion Examples 1, 2, 3 and 4 are prepared according to the following protocol:**

2.2 g of polymer are dissolved with stirring for 1 hour in 37 grams of deionized water, the pH of which is adjusted to 5 with the appropriate amount of citric acid. 9 grams of water are introduced into 50 grams of a 40% aqueous dispersion of hydrophilic $TiO_2$ nanoparticles (Mirasun TiW 60) and the pH is adjusted to 5 with the appropriate amount of citric acid. The two preceding aqueous phases are mixed by simple stirring for 30 minutes.

**2) The stability of the aqueous dispersions obtained is evaluated by measuring their viscosity according to the following test procedure:**

The viscosity measurements are carried out 24 hours after the preparation of the dispersions using a Haake RS150 rheometer equipped with cone/plate geometry (35 mm, 2°) and with a thermostatically-controlled bath in order to control the temperature. The measurements are carried out in the flow mode, the stress being varied between 0.001 and 1 Pa according to 15 stationary phases of 120 s distributed logarithmically. The value of the viscosity for a shear rate of 10 s$^{-1}$ is then measured. The lower the viscosity, the better the state of dispersion of the nanoparticles in the aqueous phase.

| Examples | Polymer | Viscosity (Pa·s) at a shear rate of 10 s$^{-1}$ |
|---|---|---|
| 1 (control) | None | 0.01 |
| 2 (outside the invention) | A | 0.03 |
| 3 (outside the invention) | B | 0.11 |
| **4 (invention)** | **C** | **0.004** |

Only the use of the polyvinylpyrrolidone C according to the invention made it possible to improve the stability of the aqueous dispersion.

**Examples 5 and 6: Aqueous dispersions comprising 10% as AM (active material) of titanium oxide (Mirasun TiW 60) in the presence of salt and at pH 5**

The dispersions are prepared according to the same procedure as for Example 1 and the rheological measurements are also carried out in the same way. The state of dispersion is also evaluated by microscopic observation.

| Examples | Ionic strength (mol/l) | Viscosity (Pa·s) at a shear rate of 10 s$^{-1}$ |
|---|---|---|
| 5 (invention) | 0 | 0.002 |
| 6 (outside the invention) | 0.17 | 0.015 |

The aqueous dispersion 6, the ionic strength of which is equal to 0.17 mol/l, is less stable than the aqueous dispersion 5, the ionic strength of which is zero.

**Examples 7 and 8: Sun milks**

| Ingredients | Example 7 (outside the invention) | Example 8 (invention) |
|---|---|---|
| Octocrylene | 8 | 8 |
| Butylmethoxydibenzoylmethane | 2 | 2 |
| $C_{12-15}$ Alkyl benzoate | 4 | 4 |
| Cyclohexadimethylsiloxane | 10 | 10 |
| Ethanol | 4 | 4 |
| Diglycol/CHDM/Isophthalates/ SIP copolymer | 2 | 2 |
| Glycerol | 2 | 2 |
| Triethanolamine | 0.3 | 0.3 |
| Titanium oxide, "Mirasun TiW 60", from Rhodia | 4 (AM) | 4 (AM) |

(continued)

| Ingredients | Example 7 (outside the invention) | Example 8 (invention) |
|---|---|---|
| Polyvinylpyrrolidone with a mass of 2500 g/mol, supplied under the name of "Luviskol K17 Powder" from BASF | 0 | 0.44 |
| Citric acid, pH 6.9 | 0.1 | 0.1 |
| Deionized water | q.s. for 100 | q.s. for 100 |

## 1) Method of preparation of the two sun milks

The emulsion is prepared by rapid introduction of the aqueous phase into the oily phase with stirring using a homogenizer of Moritz type at a stirring speed of 3000 rpm over 15 minutes. The emulsion is then cooled to ambient temperature and then subjected to strong shearing using a homogenizer of Rannie type under a pressure of 600 bar. A white and fluid milk is obtained.

For Example 7, 10 grams of Mirasun TiW 60, the pH of which is adjusted beforehand to 7 with the appropriate amount of citric acid, are introduced into the emulsion.

For Example 8, the polyvinylpyrrolidone is dissolved, with stirring for 1 hour, in 20 grams of deionized water; 10 grams of Mirasun TiW 60, the pH of which is adjusted beforehand to 7 with the appropriate amount of citric acid, are introduced into this solution with simple stirring over 30 minutes. This solution is then introduced with stirring into the emulsion.

## 2) Measurement of the whitening on the skin

30 mg of each of the formulations are applied to an area of 4 cm x 3 cm of the inner face of the forearm and are spread using the finger with 10 rotational movements.

The area of the skin treated with each of the formulations is observed with the naked eye after application.

The whitening of the skin is also measured using a spectrocolorimeter (CM 2002 from Minolta) which makes it possible to determine the brightness B of the deposit. The reduction in the whitening is evaluated by calculating the following ratio R, expressed as percentages:

$$R = 100 \times (B_{TiO_2} - B_{TiO_2 + polymer}) / (B_{TiO_2} - B_{bare\ skin})$$

According to this test, the reduction in the whitening is regarded as significant if it is at least equal to 10%.

It is observed with the naked eye that the composition 8 according to the invention, comprising hydrophilic $TiO_2$ nanoparticles in the presence of polyvinylpyrrolidone, produces a substantially lower whitening in comparison with the composition 7, which does not comprise polyvinylpyrrolidone.

This phenomenon was confirmed by the measurements of the brightness and the calculation of the reduction in the whitening.

| Compositions | R (%) |
|---|---|
| Control: Bare skin | / |
| Example 7 (outside the invention) | / |
| **Example 8 (invention)** | **45.7** |

**Examples 9 to 16: Aqueous dispersions comprising 10% as AM of titanium oxide nanoparticles treated or not treated at the surface with a polyvinylpyrrolidone**

The TiO$_2$ nanoparticles used are:

- "Mirasun TiW 60", supplied by Rhodia as hydrophilic titanium oxide nanoparticles (alumina and silica coating);
- "UV Titan M 262", supplied by Kemira as hydrophobic titanium oxide nanoparticles (PDMS coating).

The polyvinylpyrrolidones used are:

- "Luviskol K17 Powder" (number-average molar mass 2500 g/mol),
- "Kollidon 12 PF" (number-average molar mass 1300 g/mol), supplied by BASF,
- "Polyvinylpyrrolidone K 60 solution" (number-average molar mass 160 000 g/mol), supplied by Fluka.

The following eight examples of aqueous dispersions of TiO$_2$ nanoparticles were prepared:

| Compositions | TiO$_2$ used | PVP used |
|---|---|---|
| **Example 9 (outside the invention)** | Mirasun TiW 60 (hydrophilic) | None |
| **Example 10 (invention)** | Mirasun TiW 60 (hydrophilic) | Luviskol K17 Powder MW: 2500 |
| **Example 11 (invention)** | Mirasun TiW 60 (hydrophilic) | Kollidon 12 PF MW: 1300 |
| **Example 12 (invention)** | Mirasun TiW 60 (hydrophilic) | Polyvinylpyrrolidone K 60 Solution MW: 160 000 |
| **Example 13 (outside the invention)** | UV Titan M 262 (hydrophobic) | None |
| **Example 14 (outside the invention)** | UV Titan M 262 (hydrophobic) | Luviskol K17 Powder MW: 2500 |
| **Example 15 (outside the invention)** | UV Titan M 262 (hydrophobic) | Kollidon 12 PF MW: 1300 |
| **Example 16 (outside the invention)** | UV Titan M 262 (hydrophobic) | Polyvinylpyrrolidone K 60 Solution MW: 160 000 |

**1) Method of preparation of the TiO$_2$ powders treated with a polyvinylpyrrolidone**

100 ml of a 1% aqueous polyvinylpyrrolidone (PVP) solution are prepared by dissolution of 1 gram of PVP in 99 grams of deionized water at 25°C for 2 hours with mechanical stirring. 100 ml of a 10% as AM suspension of nano titanium oxide are prepared by dilution with water, the pH being adjusted to 7 using the appropriate amount of citric acid. The preceding 200 ml are mixed and milled for 48 hours using a bead mill. The aqueous phase is then evaporated under reduced pressure until a fine white powder is obtained.

**2) Method of preparation of the aqueous dispersion Examples Nos. 9 to 16**

10 grams of powder are dispersed in 90 grams of a 1% aqueous Carbopol 980 (Noveon) gel at pH 7; the dispersion is produced using an Ultra-Turrax mixer equipped with the 25F rod for 10 minutes at a speed of 24 000 rpm.

### 3) Microscopic characterization of these aqueous dispersions

For each of Examples 9 to 16, the state of dispersion of the titanium oxide nanoparticles was evaluated by microscopic observation.

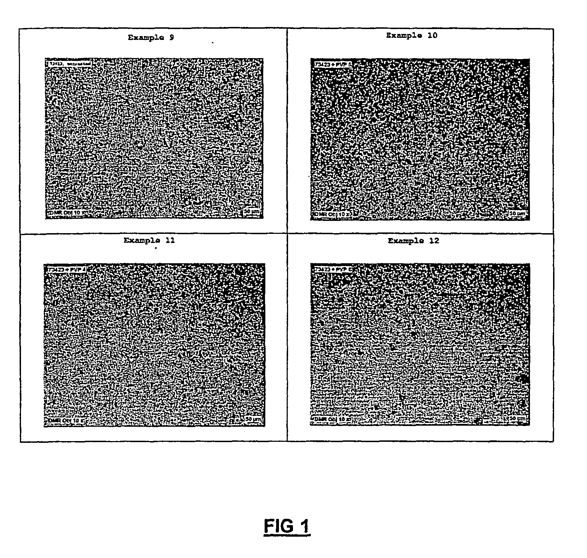

The photographs of the aqueous dispersion Examples 9 to 12 observed are represented in Figure 1 (FIG 1).

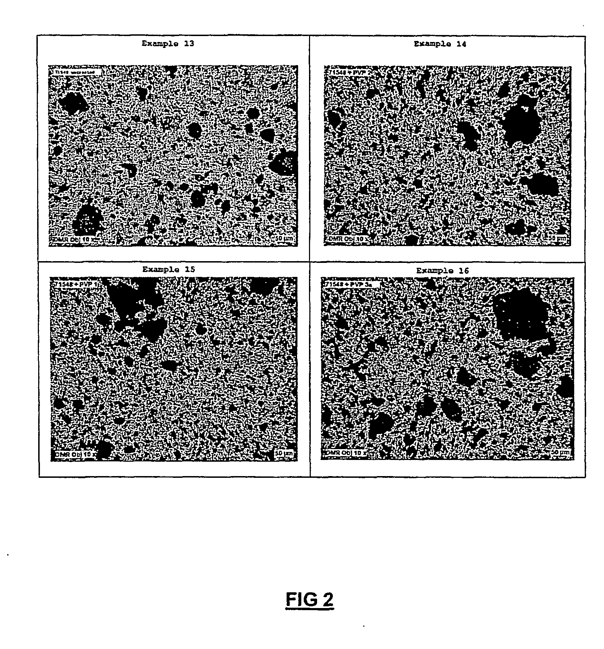

The photographs of the dispersion Examples 13 to 16 observed are represented in Figure 2 (FIG 2).

In Figure 1, it is found that, generally, the introduction of a polyvinylpyrrolidone improves the state of dispersion of the hydrophilic titanium oxide nanoparticles (Examples 10, 11 and 12, in comparison with the control Example 9 not comprising PVP).

In contrast, it is found, in Figure 2, that the addition of the polyvinylpyrrolidone has no influence on the state of dispersion of the hydrophobic titanium oxide nanoparticles (Examples 14, 15 and 16 with the control Example 13 not comprising PVP).

Furthermore, the photographs of Examples 10, 11 and 12 according to the invention show that the state of dispersion of the hydrophilic $TiO_2$ nanoparticles with the addition of the polymers "Luviskol K17 Powder" and "Kollidon 12 PF" having a molecular mass of less than 20 000 g/mol is better than that obtained with the polymer "Polyvinylpyrrolidone K 60 Solution" with a molecular mass of 160 000 g/mol.

## Claims

1. Photoprotective composition, **characterized in that** it comprises, in a physiologically acceptable medium:

   a) at least one aqueous phase,
   b) at least hydrophilic inorganic nanopigments based on metal oxides untreated with an aluminium phosphate, the said nanoparticles having a mean size for the unit particle of less than 500 nm and preferably of less than 100 nm and being treated with at least one coating agent capable of rendering them hydrophilic,
   c) at least one vinylpyrrolidone homopolymer.

2. Composition according to Claim 1, where the hydrophilic metal oxide nanoparticles have a mean size for the unit particle of between 5 nm and 500 nm, preferably between 10 nm and 100 nm and preferentially between 15 nm and 50 nm.

3. Composition according to any one of Claims 1 and 2, where the metal oxides forming these nanoparticles are chosen from titanium oxides, zinc oxides or their mixtures.

4. Composition according to Claim **3** where the coating agent is chosen from amino acids, $C_1$-$C_5$ alkanolamines, silicon oxides, metal oxides, sodium hexametaphosphate, glycerol or their mixtures.

5. Composition according to any one of Claims 1 to 4, where the hydrophilic metal oxide nanoparticles are titanium oxide nanoparticles, whether amorphous or in crystalline rutile and/or anatase form

6. Composition according to any one of Claims 1 to 5, where the hydrophilic metal oxide nanoparticles are present in proportions ranging from 0.5 to 30% by weight, with respect to the total weight of the composition, and preferably ranging from 1 to 25% by weight, with respect to the total weight of the composition.

7. Composition according to any one of Claims 1 to 6, where the vinylpyrrolidone homopolymer has a molar mass of less than 20 000 g/mol and better still of less than 10 000 g/mol.

8. Composition according to any one of Claims 1 to 7, where the concentration of vinylpyrrolidone homopolymer preferably varies from 0.01% to 10%, more preferably from 0.1% to 5% and better still from 0.2% to 2.5%.

9. Composition according to any one of Claims 1 to 8, where the ratio by weight of the metal oxide nanoparticles to the polyvinylpyrrolidone preferably varies from 1 to 30, more preferably from 5 to 20 and particularly from 8 to 12.

10. Composition according to any one of Claims 1 to 9, where the metal oxide nanoparticles are pretreated with the vinylpyrrolidone hompolymer before the introduction into a composition.

11. Composition according to any one of Claims 1 to 10, where the ionic strength of the aqueous phase of the composition, without the metal oxide, is less than 0.1 mol/l.

12. Composition according to any one of Claims 1 to 11, furthermore comprising at least one additional organic photoprotective agent active in the UV-A and/or UV-B region which is water-soluble or fat-soluble or else insoluble in the cosmetic solvents commonly used.

13. Composition according to Claim 12, where the additional organic photoprotective agent is chosen from cinnamic derivatives; anthranilates; salicylic derivatives; dibenzoylmethane derivatives; camphor derivatives; benzophenone derivatives; β,β-diphenylacrylate derivatives; triazine derivatives; benzotriazole derivatives; benzalmalonate derivatives; benzimidazole derivatives; imidazolines; bis-benzoazolyl derivatives; p-aminobenzoic acid (PABA) derivatives; methylenebis(hydroxyphenylbenzotriazole) derivatives; benzoxazole derivatives; screening polymers and screening silicones; dimers derived from α-alkylstyrene; 4,4-diarylbutadienes; and their mixtures.

14. Composition according to Claim 13, where the additional organic photoprotective agent is chosen from:

> Ethylhexyl Methoxycinnamate,
> Ethylhexyl Salicylate,
> Homosalate,
> Octocrylene,
> Phenylbenzimidazole Sulfonic Acid,
> Benzophenone-3,
> Benzophenone-4,
> Benzophenone-5,
> n-Hexyl 2-(4-diethylamino-2-hydroxybenzoyl)benzoate,
> 4-Methylbenzylidene camphor,
> Terephthalylidene Dicamphor Sulfonic Acid,
> Disodium Phenyl Dibenzimidazole Tetrasulfonate,
> Methylene Bis-benzotriazolyl Tetramethylbutylphenol,
> Bis-Ethylhexyloxyphenol Methoxyphenyl Triazine,
> Ethylhexyl triazone,
> Diethylhexyl Butamido Triazone,
> 2,4,6-Tris(dineopentyl 4'-aminobenzalmalonate)-s-triazine,
> 2,4,6-Tris(diisobutyl 4'-aminobenzalmalonate)-s-triazine,
> Drometrizole Trisiloxane,
> Polysilicone-15,
> 1,1-Dicarboxy(2,2'-dimethylpropyl)-4,4-diphenyl-butadiene,
> 2,4-Bis[5-1(dimethylpropyl)benzoxazol-2-yl-(4-phenyl)-imino]-6-(2-ethylhexyl)imino-1,3,5-triazine,
> and their mixtures.

15. Composition according to any one of Claims 12 to 14, where the additional organic photoprotective agent is present in proportions ranging from 0.01 to 20% by weight, with respect to the total weight of the composition, and preferably ranging from 0.1 to 10% by weight, with respect to the total weight of the composition.

16. Composition according to any one of Claims 1 to 15, additionally comprising at least one cosmetic adjuvant chosen from fatty substances, organic solvents, ionic or nonionic and hydrophilic or lipophilic thickening agents, softening agents, humectants, opacifiers, stabilizers, emollients, silicones, antifoaming agents, fragrances, preservatives, anionic, cationic, nonionic, zwitterionic or amphoteric surfactants, active principles, fillers, polymers, propellants, or basifying or acidifying agents.

17. Composition according to any one of Claims 1 to 16, **characterized in that** it is provided in the form of a simple or complex emulsion; in the form of an aqueous gel; or in the form of a lotion.

18. Composition according to Claim 17, **characterized in that** it is provided in the form of an oil-in-water or water-in-oil emulsion.

19. Use of a composition according to any one of Claims 1 to 18 for the manufacture of products for the cosmetic treatment of the skin, lips, nails, hair, eyelashes, eyebrows and/or scalp.

**20.** Use of a composition according to any one of Claims 1 to 18 for the manufacture of a care and/or antisun protection product for the face and/or body.

**21.** Use of a composition according to any one of Claims 1 to 18 for the manufacture of a make-up product.

**22.** Use of at least one vinylpyrrolidone homopolymer as defined in the preceding claims in a photoprotective composition comprising at least one aqueous phase and at least hydrophilic metal oxide nanoparticles untreated with an aluminium phosphate as are defined in the preceding claims for the purpose of reducing the whitening and/or of improving the stability of the said composition.

**Patentansprüche**

**1.** Lichtschutzzusammensetzung,
**dadurch gekennzeichnet, dass** sie in einem physiologisch akzeptablen Medium enthält:

a) mindestens eine wässerige Phase,
b) zumindest hydrophile anorganische Nanopigmente auf der Basis von nicht mit einem Aluminiumphosphat behandelten Metalloxiden,
wobei die Nanopartikel eine mittlere Größe des Einheitspartikels von weniger als 500 nm und vorzugsweise weniger als 100 nm aufweisen und mit mindestens einem Beschichtungsmittel behandelt sind, das befähigt ist, sie hydrophil zu machen,
und
c) mindestens ein Vinylpyrrolidon-Homopolymer.

**2.** Zusammensetzung nach Anspruch 1, bei der die hydrophilen Metalloxid-Nanopartikel eine mittlere Größe des Einheitspartikels von 5 bis 500 nm, bevorzugt von 10 bis 100 nm und noch bevorzugter von 15 bis 50 nm aufweisen.

**3.** Zusammensetzung nach einem der Ansprüche 1 und 2, bei der die Metalloxide, welche diese Nanopartikel bilden, unter Titanoxid, Zinkoxid oder ihren Gemischen ausgewählt sind.

**4.** Zusammensetzung nach Anspruch 3, bei der das Beschichtungsmittel unter Aminosäuren, $C_1$-$C_5$-Alkanolaminen, Siliciumoxiden, Metalloxiden, Natriumhexametaphosphat, Glycerin oder ihren Gemischen ausgewählt ist.

**5.** Zusammensetzung nach einem der Ansprüche 1 bis 4, bei der die hydrophilen Metalloxid-Nanopartikel Titanoxid-Nanopartikel entweder in amorpher oder in kristalliner Rutil- und/oder Anatas-Form sind.

**6.** Zusammensetzung nach einem der Ansprüche -1 bis 5, bei der die hydrophilen Metalloxid-Nanopartikel in Mengenanteilen im Bereich von 0,5 bis 30 Gew.-%, bezogen auf das Gesamtgewicht der Zusammensetzung, und vorzugsweise im Mengenbereich von 1 bis 25 Gew.-%, vorliegen, bezogen auf das Gesamtgewicht der Zusammensetzung.

**7.** Zusammensetzung nach einem der Ansprüche 1 bis 6, bei der das Vinylpyrrolidon-Homopolymer eine Molmasse von weniger als 20 000 g/mol und noch besser von weniger als 10 000 g/mol aufweist.

**8.** Zusammensetzung nach einem der Ansprüche 1 bis 7, bei der die Konzentration des Vinylpyrrolidon-Homopolymers vorzugsweise von 0,01 bis 10 %, noch bevorzugter von 0,1 bis 5 % und noch besser von 0,2 bis 2,5 % variiert.

**9.** Zusammensetzung nach einem der Ansprüche 1 bis 8, bei der das Gewichtsverhältnis der Metalloxid-Nanopartikel zum Polyvinylpyrrolidon vorzugsweise von 1 bis 30, noch bevorzugter von 5 bis 20 und insbesondere von 8 bis 12 variiert.

**10.** Zusammensetzung nach einem der Ansprüche 1 bis 9, bei der die Metalloxid-Nanopartikel vor dem Einbringen in eine Zusammensetzung mit dem Vinylpyrrolidon-Homopolymer vorbehandelt sind.

**11.** Zusammensetzung nach einem der Ansprüche 1 bis 10, bei der die Ionenstärke der wässerigen Phase der Zusammensetzung, ohne das Metalloxid, weniger als 0,1 mol/l beträgt.

**12.** Zusammensetzung nach einem der Ansprüche 1 bis 11, die ferner mindestens ein zusätzliches, im UV-A- und/oder UV-B-Bereich wirksames organisches Lichtschutzmittel enthält, das wasserlöslich oder fettlöslich ist oder auch in den üblicherweise verwendeten kosmetischen Lösungsmitteln unlöslich ist.

**13.** Zusammensetzung nach Anspruch 12, bei der das zusätzliche organische Lichtschutzmittel ausgewählt ist unter Zimtsäurederivaten; Anthranilaten; Salicylsäurederivaten; Dibenzoylmethanderivaten; Campherderivaten; Benzophenonderivaten; β,β-Diphenylacrylat-Derivaten; Triazinderivaten; Benzotriazolderivaten; Benzalmalonat-Derivaten; Benzimidazolderivaten; Imidazolinen; Bis-benzoazolylderivaten; p-Aminobenzoesäure (PABA)-Derivaten; Methylen-bis(hydroxyphenylbenzotriazol)-Derivaten; Benzoxazolderivaten; filternden Polymeren und Siliconen; von α-Alkylstyrolen abgeleiteten Dimeren und 4,4-Diarylbutadienen sowie Gemischen dieser Verbindungen.

**14.** Zusammensetzung nach Anspruch 13, bei der das zusätzliche organische Lichtschutzmittel ausgewählt ist unter:

Ethylhexyl Methoxycinnamate,
Ethylhexyl Salicylate,
Homosalate,
Octocrylene,
Phenylbenzimidazole Sulfonic Acid,
Benzophenon-3,
Benzophenon-4,
Benzophenon-5,
n-Hexyl-2-(4-diethylamino-2-hydroxybenzoyl)-benzoat,
4-Methylbenzylidencampher,
Terephthalylidene Dicamphor Sulfonic Acid,
Disodium Phenyl Dibenzimidazole Tetrasulfonate,
Methylene Bis-benzotriazolyl Tetramethylbutylphenol,
Bis-Ethylhexyloxyphenol Methoxyphenyl Triazine,
Ethylhexyl triazone,
Diethylhexyl Butamido Triazone,
2,4,6-Tris(dineopentyl-4'-aminobenzalmalonat)-s-triazin,
2,4,6-Tris(diisobutyl-4'-aminobenzalmalonat)-s-triazin,
Drometrizole Trisiloxane,
Polysilicone-15,
1, 1-Dicarboxy-(2,2'-dimethylpropyl)-4,4-diphenylbutadien und
2,4-Bis[5-1(dimethylpropyl)-benzoxazol-2-yl-(4-phenyl)-imino]-6-(2-ethylhexyl)-imino-1,3,5-triazin
sowie Gemischen dieser Verbindungen.

**15.** Zusammensetzung nach einem der Ansprüche 12 bis 14, bei der das zusätzliche organische Lichtschutzmittel in Mengenanteilen im Bereich von 0,01 bis 20 Gew.-%, bezogen auf das Gesamtgewicht der Zusammensetzung, und vorzugsweise im Mengenanteil von 0,1 bis 10 Gew.-% vorliegt, bezogen auf das Gesamtgewicht der Zusammensetzung.

**16.** Zusammensetzung nach einem der Ansprüche 1 bis 15, die zusätzlich mindestens ein kosmetisches Adjuvans enthält, das ausgewählt ist unter Fettsubstanzen, organischen Lösungsmitteln, ionischen oder nichtionischen und hydrophilen oder lipophilen Verdickungsmitteln, Erweichungsmitteln, Befeuchtungsmitteln, Trübungsmitteln, Stabilisatoren, Emollientien, Siliconen, Antischaummitteln, Duftstoffen, Konservierungsmitteln, anionischen, kationischen, nichtionischen, zwitterionischen oder amphoteren Tensiden, Wirkstoffen, Füllstoffen, Polymeren, Treibmitteln oder Mitteln zum Basischmachen oder zum Ansäuern.

**17.** Zusammensetzung nach einem der Ansprüche 1 bis 16, **dadurch gekennzeichnet, dass** sie in Form einer einfachen oder komplexen Emulsion, in Form eines wässerigen Gels oder in Form einer Lotion vorliegt.

**18.** Zusammensetzung nach Anspruch 17, **dadurch gekennzeichnet, dass** sie in Form einer Öl-in-Wasser-Emulsion oder einer Wasser-in-Öl-Emulsion vorliegt.

**19.** Verwendung einer Zusammensetzung nach einem der Ansprüche 1 bis 18 zur Herstellung von Produkten zur kosmetischen Behandlung der Haut, der Lippen, der Nägel, des Haars, der Wimpern, der Augenbrauen und/oder der Kopfhaut.

**20.** Verwendung einer Zusammensetzung nach einem der Ansprüche 1 bis 18 zur Herstellung eines Pflegeprodukts und/oder eines Sonnenschutzprodukts für das Gesicht und/oder den Körper.

**21.** Verwendung einer Zusammensetzung nach einem der Ansprüche 1 bis 18 zur Herstellung eines Make-up-Produkts.

**22.** Verwendung mindestens eines Vinylpyrrolidon-Homopolymers wie in den vorhergehenden Ansprüchen definiert in einer Lichtschutzzusammensetzung, die mindestens eine wässerige Phase und zumindest hydrophile Metalloxid-Nanopartikel enthält, die nicht mit einem Aluminiumphosphat behandelt sind, wie in den vorhergehenden Ansprüchen definiert, zur Verringerung des Weißmacheffekts und/oder zur Verbesserung der Stabilität der Zusammensetzung.

**Revendications**

**1.** Composition photoprotectrice **caractérisée en ce qu'**elle comprend, dans un milieu physiologiquement acceptable :

   a) au moins une phase aqueuse,
   b) au moins des nanopigments inorganiques hydrophiles à base d'oxydes métalliques non traités avec un phosphate d'aluminium, lesdites nanoparticules présentant une taille moyenne pour la particule unitaire inférieure à 500 nm et, de préférence, inférieure à 100 nm, et étant traitées avec au moins un agent de revêtement capable de les rendre hydrophiles,
   c) au moins un homopolymère de vinylpyrrolidone.

**2.** Composition selon la revendication 1, dans laquelle les nanoparticules d'oxydes métalliques hydrophiles présentent une taille moyenne pour la particule unitaire comprise entre 5 nm et 500 nm, de préférence, entre 10 nm et 100 nm et, préférentiellement, entre 15 nm et 50 nm.

**3.** Composition selon l'une quelconque des revendications 1 et 2, dans laquelle les oxydes métalliques formant ces nanoparticules sont choisis parmi des oxydes de titane, des oxydes de zinc ou leurs mélanges.

**4.** Composition selon la revendication 3, dans laquelle l'agent de revêtement est choisi parmi des acides aminés, des alcanolamines en $C_1$-$C_5$, des oxydes de silicium, des oxydes métalliques, l'hexamétaphosphate de sodium, le glycérol ou leurs mélanges.

**5.** Composition selon l'une quelconque des revendications 1 à 4, dans laquelle les nanoparticules d'oxydes métalliques hydrophiles sont des nanoparticules d'oxyde de titane, qu'elles soient amorphes ou sous une forme cristalline rutile et/ou anatase.

**6.** Composition selon l'une quelconque des revendications 1 à 5, dans laquelle les nanoparticules d'oxydes métalliques hydrophiles sont présentes en des proportions s'échelonnant de 0,5 à 30 % en poids par rapport au poids total de la composition et, de préférence, s'échelonnant de 1 à 25 % en poids par rapport au poids total de la composition.

**7.** Composition selon l'une quelconque des revendications 1 à 6, dans laquelle l'homopolymère de vinylpyrrolidone présente une masse molaire inférieure à 20 000 g/mol et, encore mieux, inférieure à 10 000 g/mol.

**8.** Composition selon l'une quelconque des revendications 1 à 7, dans laquelle la concentration en homopolymère de vinylpyrrolidone varie, de préférence, de 0,01 % à 10 %, plus préférablement, de 0,1 % à 5 % et, encore mieux, de 0,2 % à 2,5 %.

**9.** Composition selon l'une quelconque des revendications 1 à 8, dans laquelle le rapport pondéral entre les nanoparticules d'oxydes métalliques et la polyvinylpyrrolidine varie, de préférence, de 1 à 30, plus préférablement, de 5 à 20 et, en particulier, de 8 à 12.

**10.** Composition selon l'une quelconque des revendications 1 à 9, dans laquelle les nanoparticules d'oxydes métalliques sont prétraitées avec l'homopolymère de vinylpyrrolidone avant l'introduction dans une composition.

**11.** Composition selon l'une quelconque des revendications 1 à 10, dans laquelle la force ionique de la phase aqueuse de la composition, sans l'oxyde métallique, est inférieure à 0,1 mol/l.

**12.** Composition selon l'une quelconque des revendications 1 à 11, comprenant, en outre, au moins un agent photoprotecteur organique supplémentaire, actif dans la région UV-A et/ou UV-B, qui est hydrosoluble ou oléosoluble, ou bien insoluble dans les solvants cosmétiques couramment utilisés.

**13.** Composition selon la revendication 12, dans laquelle l'agent photoprotecteur organique supplémentaire est choisi parmi des dérivés cinnamiques ; des anthranilates ; des dérivés salicyliques ; des dérivés du dibenzoylméthane ; des dérivés du camphre ; des dérivés de la benzophénone ; des dérivés de l'acrylate de ß,ß-diphényle, des dérivés de la triazine ; des dérivés du benzotriazole ; des dérivés du benzalmalonate ; des dérivés du benzimidazole ; des imidazolines ; des dérivés de bis-benzoazolyle ; des dérivés de l'acide p-aminobenzoïque (PABA) ; des dérivés du méthylène-bis(hydroxyphénylbenzotriazole) ; des dérivés du benzoxazole ; des polymères photoprotecteurs et des silicones photoprotectrices ; des dimères dérivés d'un a-alkylstyrène ; des 4,4-diarylbutadiènes ; et leurs mélanges.

**14.** Composition selon la revendication 13, dans laquelle l'agent photoprotecteur organique supplémentaire est choisi parmi :

le méthoxycinnamate d'éthylhexyle,
le salicylate d'éthylhexyle,
l'homosalate,
l'octocrylène,
l'acide phénylbenzimidazole sulfonique,
la benzophénone-3,
la benzophénone-4,
la benzophénone-5,
le 2-(4-diéthylamino-2-hydroxybenzoyl)benzoate de n-hexyle,
le 4-méthylbenzylidène-camphre,
l'acide téréphtalylidène-dicamphre sulfonique,
le tétrasulfonate de phényl-dibenzimidazole disodique,
le méthylène-bis-benzotriazolyl-tétraméthylbutyl-phénol,
la bis-éthylhexyloxyphénol-méthoxyphényl-triazine,
l'éthylhexyl-triazone,
la diéthylhexylbutamido-triazone,
la 2,4,6-tris(dinéopentyl-4'-aminobenzalmalonate)-s-triazine,
la 2,4,6-tris(diisobutyl-4'-aminobenzalmalonate)-s-triazine,
le drométrizole-trisiloxane,
la polysilicone-15,
le 1,1-dicarboxy(2,2'-diméthylpropyl)-4,4-diphényl-butadiène,
la 2,4-bis[5-1(diméthylpropyl)benzoxazol-2-yl-(4-phényl)imino]-6-(2-éthylhexyl)imino-1,3,5-triazine,
et leurs mélanges.

**15.** Composition selon l'une quelconque des revendications 12 à 14, dans laquelle l'agent photoprotecteur organique supplémentaire est présent en des proportions s'échelonnant de 0,01 à 20 % en poids, par rapport au poids total de la composition, et s'échelonnant, de préférence, de 0,1 à 10 % en poids, par rapport au poids total de la composition.

**16.** Composition selon l'une quelconque des revendications 1 à 15, comprenant, en outre, au moins un adjuvant cosmétique choisi parmi des substances grasses, des solvants organiques, des agents épaississants ioniques ou non ioniques et hydrophiles ou lipophiles, des agents adoucissants, des agents humectants, des agents opacifiants, des agents stabilisants, des agents émollients, des silicones, des agents anti-mousse, des parfums, des conservateurs, des agents tensioactifs anioniques, cationiques, non ioniques, zwitterioniques ou amphotères, des principes actifs, des charges, des polymères, des propulseurs, ou des agents alcalinisants ou acidifiants.

**17.** Composition selon l'une quelconque des revendications 1 à 16, **caractérisée en ce qu'**elle est procurée sous la forme d'une émulsion simple ou complexe ; sous la forme d'un gel aqueux ; ou sous la forme d'une lotion.

**18.** Composition selon la revendication 17, **caractérisée en ce qu'**elle est procurée sous la forme d'une émulsion huile/eau ou eau/huile.

**19.** Utilisation d'une composition selon l'une quelconque des revendications 1 à 18, pour la fabrication de produits destinés au traitement cosmétique de la peau, des lèvres, des ongles, des cheveux, des cils, des sourcils et/ou du cuir chevelu.

**20.** Utilisation d'une composition selon l'une quelconque des revendications 1 à 18, pour la fabrication d'un produit de soins et/ou de protection antisolaire pour le visage et/ou le corps.

**21.** Utilisation d'une composition selon l'une quelconque des revendications 1 à 18, pour la fabrication d'un produit de maquillage.

**22.** Utilisation d'au moins un homopolymère de vinylpyrrolidone, tel que défini dans les revendications précédentes, dans une composition photoprotectrice comprenant au moins une phase aqueuse et au moins des nanoparticules d'oxydes métalliques hydrophiles non traitées avec un phosphate d'aluminium, telles qu'elles sont définies dans les revendications précédentes, dans le but de réduire le blanchiment et/ou d'améliorer la stabilité de ladite composition.

Example 9 | Example 10
Example 11 | Example 12

**FIG 1**

**FIG 2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001048731 A
- WO 02055046 A
- WO 2004104111 A
- EP 669323 A
- US 2463264 A
- US 5237071 A
- US 5166355 A
- GB 2303549 A
- DE 19726184
- EP 893119 A
- EP 0832642 A
- EP 1027883 A
- EP 1300137 A
- DE 10162844
- WO 9304665 A
- DE 19855649
- EP 0967200 A
- DE 19746654
- DE 19755649
- EP 1008586 A
- EP 1133980 A
- EP 133981 A
- WO 9206778 A
- FR 2315991
- FR 2416008
- US 4077441 A
- US 4850517 A

**Non-patent literature cited in the description**

- *Cosmetics & Toiletries,* February 1990, vol. 105, 53-64
- **STANDISH ; WATKINS.** *J. Mol. Biol.,* 1965, vol. 13, 238